# DEMANDE DE BREVET EUROPEEN

(11) **EP 1 350 465 A1**
(43) Date de publication de la demande: **08.10.2003**
(21) Numéro de dépôt: 03356052.5
(22) Date de dépôt: 20.03.2003
(51) Int. Cl.: A61B 5/103

(54) **Procédé de mesure concernant la rigidité d'un pénis et dispositif pour la mise en oeuvre de ce procédé**

(30) Priorité: 02.04.2002 FR 0204073
(71) Demandeur: Lavoisier Pierre, 69003 LYON (FR)
(72) Inventeur: Lavoisier Pierre, 69003 LYON (FR)
(74) Mandataire: Maureau, Philippe

(57) **Abrégé**

Ce procédé permet la mesure de la pression et du débit sanguin artériel et veineux au niveau du pénis. Il comporte les étapes suivantes:
- remplissage de deux réservoirs, situés sur la face interne d'un manchon (1) avec un fluide,
- connexion d'un capteur de pression (6) au premier réservoir (3),
- remplissage d'un réservoir de grand volume (15),
- connexion du réservoir de grand volume (15) au second réservoir (11) grâce à une tubulure de pression (10),
- connexion à ladite tubulure de pression (10), d'une membrane (13) souple, vis-à-vis de laquelle une sonde de photopléthysmographie (14) est positionnée,
- mise en place du manchon (1) autour du pénis du patient,
- fermeture du manchon (1),
- mesure de la pression du fluide à l'intérieur du premier réservoir (3),
- émission d'un rayon lumineux par la sonde vers la membrane et mesure par celle-ci du rayon lumineux réfléchi,
- amplification puis enregistrement et/ou affichage des mesures effectuées.

L'invention comprend également un dispositif pour mettre en oeuvre le procédé de mesure ci-dessus.

## Description

La présente invention concerne un procédé de mesure concernant l'érection d'un pénis, ainsi qu'un dispositif pour la mise en oeuvre de ce procédé. Ces mesures sont effectuées pour la détermination d'un diagnostic dans le domaine des dysfonctions érectiles. Elles sont faites au cours du sommeil du patient, car durant la phase de sommeil paradoxal, chaque homme normal a des érections, mais elles peuvent également être effectuées le jour pour le diagnostic et le traitement.

Il est connu de mesurer l'érection nocturne du pénis, afin de pouvoir dissocier les impuissances organiques des impuissances psychogènes. Les patients ayant une impuissance organique n'ont pas d'érection de jour, ni durant le sommeil paradoxal, tandis que les patients psychogènes ont des érections au cours du sommeil, mais n'en ont pas le jour.

Lors d'une érection, le corps caverneux se remplit de sang. Dans une première phase, son volume augmente jusqu'à atteindre son volume maximal: c'est la tumescence, ce qui correspond à la pression infrasystolique. Dans une seconde phase, la pression intérieure augmente: c'est la rigidité, et la pression est suprasystolique. Le corps caverneux comporte une entrée artérielle et une sortie veineuse. Pour que la pression monte, il est nécessaire que le débit d'entrée augmente et que, simultanément, le débit de sortie diminue.

Lorsque la mesure de la pression intra caverneuse du pénis d'un patient révèle une impuissance organique, il faut déterminer si celle-ci est due ou non à une diminution du débit d'entrée (pathologie artérielle) ou à une absence de diminution du débit de sortie, ceci permettra de déterminer l'origine de l'impuissance organique constatée.

Dans les méthodes connues de la mesure de rigidité pénienne - comme celle décrite dans la demande de brevet français FR 2726457 -, au moins un manchon est fixé autour du pénis du patient. Ce manchon permet de mesurer deux paramètres qui sont ensuite utilisés pour calculer les flux sanguins entrant et sortant des corps caverneux et déterminer si il y a ou non une fuite veineuse.

D'abord on mesure la pression intra caverneuse à l'aide d'un capteur de pression relié à un réservoir placé entre un manchon pénien et le pénis du patient.

Ensuite, on mesure les variations de débit sanguin artériel pénien à l'aide d'une sonde de photopléthysmographie reliée au réservoir (ou à un autre réservoir) placé entre le manchon et le pénis du patient. Dans les procédés connus, la sonde de photopléthysmographie est fixée de manière à être en contact avec la surface du pénis. Néanmoins pendant la mesure, la sonde pléthysmographique est en fait orientée non pas directement vers la peau, mais plutôt vers la membrane du réservoir de fluide en contact avec la peau, et elle mesure le déplacement de la membrane en fonction du pouls artériel.

Or, il est apparu que les mesures de déplacement de la membrane du réservoir fixé au manchon pénien peuvent varier. En particulier lors d'une érection, le réservoir de liquide auquel la sonde de photopléthysmographie est reliée, est sous pression variable, et les déplacements de la membrane du réservoir ont par conséquent une amplitude variable, ceci pouvant mener à des mesures faussées du débit sanguin artériel.

Ensuite, dans les dispositifs connus, le réservoir auquel la sonde de photopléthysmographie est reliée est rempli d'un gaz dont la pression est contrôlée. Or il a été trouvé qu'en pratique, les mesures sont faussées par le fait que la pression du fluide contenu dans le réservoir du manchon pénien auquel est reliée la sonde du pléthysmographe n'est pas stable. Une régulation automatique de la pression n'est pas suffisante. Dans tous les cas, des variations de volume parasites sont relevées par la sonde de photopléthysmographie, qui sont dues à la nature compressible des gaz mais également à d'autres paramètres complexes comme à des variations conjointes du débit de plusieurs artères, ou au degré variable d'oxygénation des hématies qui modifie la quantité de lumière réfléchie vers la sonde du pléthysmographe. Il est indispensable de supprimer ces variations de volumes parasites mesurées, pour obtenir un signal purifié et donc fiable.

La présente invention a pour objectif de proposer un procédé et un dispositif de mesure de l'érection d'un pénis par photopléthysmographie, ledit procédé permettant de parer aux désavantages cités plus haut.

Cet objectif est atteint avec un dispositif, qui permet de compenser les désavantages des dispositifs connus.

Premièrement la sonde de photopléthysmographie est n'est pas placée directement au contact du pénis, mais plutôt à distance du manchon pénien grâce à une membrane très fine et souple de type latex, ladite membrane venant obturer une extrémité d'un tube rempli d'un fluide incompressible, l'autre extrémité étant connectée au réservoir situé à la face interne du manchon pénien par l'intermédiaire d'un raccord à trois voies, dont la troisième voie est reliée à un réservoir de grand volume du fluide incompressible, de sorte que la pression de ce fluide à l'intérieur du dispositif est à peu près constante. En outre les variations de pression du fluide à l'intérieur du second réservoir sont de préférence contrôlées par un capteur de pression et enregistrées. Grâce à la membrane de latex, la sonde photopléthysmographique mesure les variations de déplacement induites par les variations de volume dans le second réservoir, qui elles-mêmes sont dues au pouls artériel dans le corps caverneux. Ces variations de volume peuvent être très faibles, mais la souplesse de la membrane de latex et le fait que le déplacement de cette membrane n'est pas entravé, autorisent des mesures du pouls artériel très fines.

Deuxièmement, dans le dispositif de mesure selon l'invention, le réservoir auquel est relié la sonde photopléthysmographique est rempli d'un fluide incompressible, par exemple de l'eau, ce qui permet de s'affranchir des variations de volume causées par la nature compressible des gaz.

Les mesures de pression intra caverneuse sont réalisées comme dans les dispositifs connus, à l'aide d'un manchon rempli de liquide auquel est relié un capteur de pression.

L'invention fournit également un procédé pour obtenir la courbe des variations de débit sanguin artériel et veineux qui comporte les étapes suivantes:
- mesure de la pression intra-caverneuse par le capteur de pression.
- enregistrement du signal du pouls par la sonde de photopléthysmographie pendant la période de repos, puis de tumescence, puis de rigidité du pénis,
- évaluation du débit sanguin artériel à partir de la surface sous la courbe de cet enregistrement,

En utilisant les deux mesures ci-dessus, on peut calculer la courbe du débit sanguin veineux, en procédant selon les étapes suivantes:
- enregistrement du signal du capteur de pression pour les pressions intra-caverneuses,
- obtention de la courbe des variations de débit sanguin artériel comme exposé ci-dessus,
- calcul à partir de la courbe précédente et de la courbe des variations de débit sanguin artériel, par les lois de mécanique des fluides, des variations de débit sanguin veineux afin d'en obtenir la courbe.

Si le débit d'entrée artériel augmente, sans que la pression ni le volume n'augmentent, on peut en déduire que le débit d'entrée est égal au débit de sortie, ce qui permet d'établir qu'il n'y a pas de rétention sanguine au niveau du pénis.

Le procédé inclut, de préférence, la mesure en continu de la fréquence du pouls, de la durée et des variations de pression intra-caverneuse, ainsi que des variations de débit artériel.

Il est alors aussi possible à l'aide de ces mesures de classer chaque patient dans un groupe. Afin de permettre cette classification, l'invention propose d'effectuer en plus les étapes suivantes:
- mesure de la pression du fluide selon le procédé décrit ci-dessus par exemple au cours du sommeil d'un patient,
- tracé de la courbe obtenue,
- mise en évidence pour chaque période de tumescence d'un plateau correspondant à la phase vasculaire et de pics correspondant à la phase musculaire,
- évaluation de la surface du plateau et des pics correspondant à ce plateau,
- report de ces résultats sur un diagramme sur lequel la surface du plateau se trouve en abscisse et celle des pics en ordonnée ou inversement, et
- tracé d'un graphique où le temps est en abscisse et la pression correspondant au plateau ainsi que les pressions maximales des pics sont en ordonnée.

La présente invention propose également un dispositif pour la mise en pratique des procédés décrits ci-dessus. Ce dispositif est du type comprenant:
- un manchon constitué par une bande de matériau non élastique ayant l'une de ses faces entièrement couverte par un premier réservoir en plastique souple, destiné à être rempli par un fluide, ledit manchon comprenant également un second réservoir en plastique souple destiné à être rempli de fluide,
- des moyens pour mettre en place, fermer et maintenir fermement la bande de matériau non élastique autour du pénis d'un patient, de telle sorte qu'en position fermée, les réservoirs se situent à l'intérieur du manchon,
- un capteur de pression en communication directe avec l'intérieur du premier réservoir en plastique, pour y mesurer la pression de fluide, correspondant à la pression intra-caverneuse,
- un photopléthysmographe dont la sonde est reliée au second réservoir,
- des moyens électroniques pour modifier la gamme de sensibilité du capteur de pression et du photopléthysmographe, pour amplifier, enregistrer et/ou afficher les signaux fournis par ceux-ci,
caractérisé en ce qu'il comporte en outre:
(i) un réservoir de grand volume rempli d'un fluide incompressible du même type que celui utilisé pour remplir les premier et second réservoirs, et
(ii) une tubulure de pression reliant directement ledit réservoir de grand volume au second réservoir, et
(iii) un raccord à trois voies reliant les tubulures de pression entre le réservoir de grand volume et le second réservoir, et dont la troisième voie est obturée par une membrane très fine et souple de type en latex, vis-à-vis de laquelle la sonde de photopléthysmographie est positionnée pour les mesures du pouls artériel pénien.

De toute façon, l'invention sera bien comprise à l'aide de la description qui suit, en référence au dessin schématique annexé représentant à titre d'exemples non limitatifs, des dispositifs selon l'invention permettant la mise en oeuvre des procédés décrits ci-dessus:

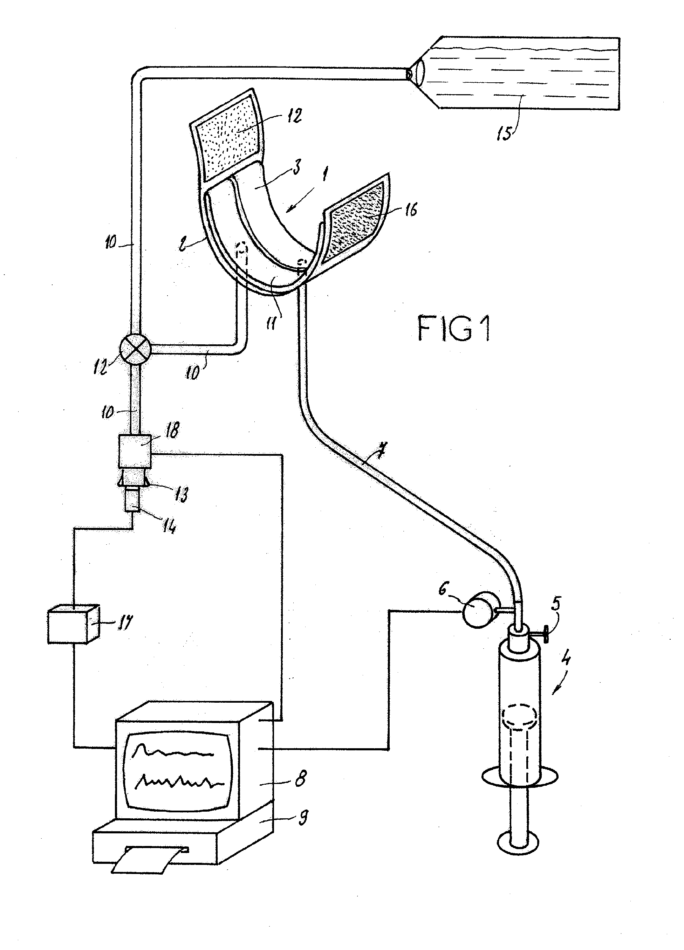

Figure 1 est une vue en perspective d'une forme de réalisation d'un dispositif selon l'invention.

Un dispositif pour mesurer la pression intra-caverneuse et le débit sanguin au niveau du pénis d'un patient est représenté à la figure 1. Il comporte un manchon 1 constitué par une bande 2 de matériau synthétique non élastique, ayant une de ses faces couverte d'un premier réservoir 3 en plastique transparent pouvant être rempli d'un fluide incompressible, de préférence un liquide, tel de l'eau, à l'aide d'une seringue 4. Un robinet trois voies 5 est monté entre la seringue 4 et le réservoir 3 afin de pouvoir retirer la seringue 4 une fois le réservoir 3 rempli. Un capteur de pression 6 mesure la pression régnant dans le réservoir 3. Une tubulure de pression 7 relie le capteur de pression 6 au réservoir 3.

Les signaux mesurés par le capteur de pression 6 sont amplifiés et transmis à un ordinateur 8, dans la mémoire duquel ces signaux amplifiés sont enregistrés et sur l'écran duquel ils sont affichés. Cet ordinateur 8 permet aussi le traitement numérique de ces signaux, afin de les analyser et de faire une étude analytique et statistique. Une imprimante 9 permet l'impression des courbes affichées à l'écran ainsi que tout les résultats numériques.

Un second réservoir 11 en matière plastique souple est placé sur une face du manchon 1 - sur la même face que le premier réservoir -. Le second réservoir 11 est relié à un raccord à trois voies 12 par une tubulure de pression 10. La première sortie du raccord à trois voies 12 est obturée par une membrane de latex 13. Une sonde de photopléthysmographie 14 est placée vis-à-vis de cette membrane de latex 13 à environ 1 mm de distance, de manière à pouvoir en mesurer les déplacements induits par le pouls artériel dans le corps caverneux. La seconde voie du raccord 12 est connectée à un réservoir de grand volume 15 tel une poche de perfusion, rempli d'eau ou autre fluide incompressible. De préférence, le réservoir de grand volume 15 contient environ 5 litres de liquide, et est disposé de telle sorte que la surface de liquide dans ce réservoir soit de l'ordre de 600 cm², de sorte que quand le volume du pénis augmente, un peu de liquide du second réservoir 11 retourne dans le réservoir de grand volume 15. Ledit réservoir de grand volume 15 est placé trente centimètres environ au-dessus du pénis du patient, de manière à maintenir constante la pression dans le second réservoir 11 et les tubulures 10 qui y sont directement connectées, quel que soit le volume du pénis.

La sonde 14 du photopléthysmographe comporte une diode photoémettrice et une cellule photoélectrique. Un rayon lumineux est envoyé par la diode en direction de la membrane de latex et le rayon réfléchi est capté par la cellule. Le signal émis par la cellule photoélectrique est amplifié par l'amplificateur, enregistré dans la mémoire de l'ordinateur 8, et/ou imprimé sur l'imprimante 9.

Avant d'utiliser ce dispositif, les premier 3 et second 11 réservoirs ainsi que les tubulures 7 et 10 et le réservoir de grand volume 15 connecté au second réservoir 11, sont remplis d'un fluide incompressible, de préférence un liquide, par exemple de l'eau.

Pour effectuer des mesures, le manchon pénien 1 est alors mis en place autour du pénis du patient (non représenté) lorsqu'il n'est pas en érection, avec les premier 3 et second 11 réservoirs en contact direct avec le pénis. Des bandes autoagrippantes 16 permettent de fermer fermement le manchon 1 autour du pénis.

Lorsque l'érection survient, la pression à l'intérieur du corps caverneux augmente. Le premier réservoir 3 rempli d'eau se trouve alors entre le pénis qui exerce sur lui une pression et la bande 2 non élastique. La pression régnant à l'intérieur du corps caverneux est transmise à l'eau du premier réservoir 3 qui est alors comprimée. Pendant une période prédéterminée, qui peut être par exemple toute une nuit, le capteur de pression 6 mesure les variations de pression, qui sont amplifiées et transmises à l'ordinateur 8 qui les analyse et les traite.

Simultanément, le photopléthysmographe 17 mesure les variations de débit sanguin artériel. En outre les variations de pression du fluide à l'intérieur du second réservoir 11 et des tubulures 10 qui y sont connectées, sont de préférence contrôlées par une sonde de pression 18 et enregistrées de manière à permettre une correction par ordinateur si de minimes variations de pression venaient à apparaître malgré la présence du réservoir de grand volume 15.

Grâce à la membrane de latex 13, la sonde photopléthysmographique 14 mesure les variations de déplacement induites par les variations de volume dans le second réservoir 11, qui elles-mêmes sont synchrones au pouls artériel dans le corps caverneux. Ces variations de volume peuvent être très faibles, mais la souplesse de la membrane de latex 13 et le fait que le déplacement de cette membrane n'est pas entravé comme c'est le cas pour la membrane du second réservoir, autorisent des mesures du pouls artériel très fines.

De plus, dans le dispositif de mesure selon l'invention, le second réservoir 11 auquel est relié la sonde photopléthysmographique 14, est rempli d'un fluide incompressible, par exemple de l'eau, ce qui permet de s'affranchir des variations de volumes causées par la nature compressible des gaz utilisés dans les dispositifs connus.

L'invention fournit alors un procédé pour obtenir la courbe des variations de débit sanguin artériel et veineux qui comporte les étapes suivantes:
a) mesure de la pression intra-caverneuse par le capteur de pression: un capteur de pression 6 enregistre la pression intra-caverneuse et produit une courbe des variations de cette pression. En utilisant ce procédé, il est possible de mettre en évidence des plateaux de pression qui correspondent à la tumescence, et des pics de pression qui sont liés aux contractions musculaires des ischio-caverneux.
   Grâce à des procédés informatiques avec analyse du signal, il est alors possible de mesurer la surface des plateaux, la surface des pics, la hauteur des pics, la différence entre pression maximale et minimale de chaque pic, la moyenne de ces différences de pression, etc. A partir de ces mesures, une analyse statistique (analyse factorielle) fait alors ressortir deux composantes principales: la surface des pics et la surface des plateaux.
b) enregistrement du signal du pouls par la sonde de photopléthysmographie pendant la période de repos, puis de tumescence, puis de rigidité du pénis,
c) évaluation du débit sanguin artériel à partir de la surface sous la courbe de cet enregistrement,

En utilisant les deux mesures ci-dessus, on peut calculer la courbe du débit sanguin veineux, en procédant selon les étapes suivantes:
- enregistrement du signal du capteur de pression pour les pressions intra-caverneuses,
- obtention de la courbe des variations de débit sanguin artériel comme exposé ci-dessus,
- calcul à partir de la courbe précédente et de la courbe des variations de débit sanguin artériel, par les lois classiques de mécanique des fluides, des variations de débit sanguin veineux afin d'en obtenir la courbe.

La connaissance des variations de débit sanguin veineux donne une information directement exploitable pour évaluer les fuites caverno-veineuses.

Afin de classer les patients dans des groupes pour permettre d'aider le médecin à faire un diagnostic, les signaux du capteur de pression 6 sont enregistrés toute une nuit au cours du sommeil du patient. La courbe correspondante est alors tracée. Comme indiqué ci-dessus, à chaque tumescence apparaissent un plateau de pression et des pics de pression dont on évalue les surfaces. Les résultats de ces évaluations sont alors portés sur un diagramme sur lequel la surface des pics est portée en abscisse et celle du plateau est portée en ordonnée. De plus, on trace un graphique avec le temps sur l'axe des abscisses et la pression en ordonnée sur lequel est alors représenté la pression et la durée du plateau de pression ainsi que la pression maximale des pics.

Quatre groupes de patients sont alors mis en évidence:
- les faibles pour lesquels les deux surfaces mesurées sont relativement petites: ils n'ont pas de tumescence;
- les moyens qui ont une tumescence réduite et une érection peu rigide;
- les patients ayant une pathologie musculaire, qui ont une tumescence satisfaisante mais avec une rigidité faible, et
- les hypers présentant une tumescence et une rigidité satisfaisantes.

## Revendications

1. Procédé de mesure concernant la rigidité d'un pénis comportant les étapes suivantes:
a) remplissage avec un fluide, avant de l'utiliser, d'un premier réservoir (3) en matière plastique souple, situé sur la face interne d'un manchon (1) constitué d'une bande d'un matériau non élastique,
b) remplissage avec un fluide, avant de l'utiliser, d'un second réservoir (11) en matière plastique souple, situé sur la face interne du manchon (1),
c) connexion d'un capteur de pression (6) au premier réservoir (3),
d) mise en place du manchon (1) autour du pénis du patient lorsqu'il n'est pas en érection, les réservoirs en plastique (3, 11) remplis de fluide étant en contact avec le pénis,
e) fermeture du manchon (1) afin de maintenir fermement les réservoirs en plastique (3, 11) remplis de fluide contre le pénis du patient,
f) mesure de la pression du fluide à l'intérieur du premier réservoir en plastique (3), cette mesure étant corrélée à la pression intra-caverneuse,
g) émission durant la mesure, d'un rayon lumineux par une sonde de photopléthysmographie (14) et mesure par celle-ci à l'aide d'une cellule photoélectrique du rayon lumineux réfléchi,
h) amplification puis enregistrement et/ou affichage des mesures effectuées,
**caractérisé en ce qu'**il comporte en outre les étapes suivantes, entre les étapes c et d ci-dessus:
(i) remplissage, avant de l'utiliser, d'un réservoir de grand volume (15) avec un fluide incompressible du même type que celui qui est utilisé pour remplir les premier (3) et second (11) réservoirs, et
(ii) connexion dudit réservoir de grand volume (15) au second réservoir (11) grâce à une tubulure de pression (10), et
(iii) connexion à ladite tubulure de pression (10), par l'intermédiaire d'un raccord à trois voies (12), d'une membrane (13) très fine et souple de type en latex, vis-à-vis de laquelle la sonde de photopléthysmographie (14) est positionnée pour les mesures du pouls artériel pénien.

2. Procédé selon la revendication 1, **caractérisé en ce qu'**il comporte en outre les étapes suivantes pour obtenir la courbe des variations de débit sanguin artériel:
- mesure de la pression intra-caverneuse par le capteur de pression (6),
- enregistrement du signal du pouls par la sonde de photopléthysmographie (14) pendant la période de repos, puis de tumescence, puis de rigidité du pénis,
- évaluation du débit sanguin artériel à partir de la surface sous la courbe de cet enregistrement.

3. Procédé selon la revendication 2, **caractérisé en ce qu'**il comporte en outre, à partir de la courbe de variations de pression intra-caverneuse et de la courbe des variations de débit sanguin artériel, le calcul par les lois de mécanique des fluides, des variations de débit sanguin veineux afin d'en obtenir la courbe.

4. Procédé selon la revendication 3, **caractérisé en ce qu'**il comporte en outre les étapes suivantes:
- mesure de la pression du fluide à l'intérieur du premier réservoir (3) selon le procédé de la revendication 1 au cours du sommeil d'un patient,
- tracé de la courbe obtenue,
- mise en évidence pour chaque période de tumescence, d'un plateau correspondant à la phase vasculaire et de pics correspondant à la phase musculaire,
- évaluation de la surface du plateau et des pics correspondant à ce plateau,
- report de ces résultats sur un diagramme sur lequel la surface du plateau se trouve en abscisse et celle des pics en ordonnée ou inversement, et
- tracé d'un graphique où le temps est en abscisse et la pression correspondant au plateau ainsi que les pressions maximales des pics sont en ordonnée.

5. Dispositif pour la mise en oeuvre du procédé selon l'une quelconque des revendications 1 à 4, du type comprenant:
- un manchon (1) constitué par une bande de matériau non élastique (2) ayant l'une de ses faces entièrement couverte par un premier réservoir (3) en plastique souple, destiné à être rempli par un fluide, ledit manchon (1) comprenant également un second réservoir (11) en plastique souple destiné à être rempli de fluide,
- des moyens (16) pour mettre en place, fermer et maintenir fermement la bande de matériau non élastique (2) autour du pénis d'un patient, de telle sorte qu'en position fermée, les réservoirs (3, 11) se situent à l'intérieur du manchon (1),
- un capteur de pression (6) en communication directe avec l'intérieur du premier réservoir (3) en plastique, pour y mesurer la pression de fluide, correspondant à la pression intra-caverneuse,
- un photopléthysmographe (17) dont la sonde (14) est reliée au second réservoir (11),
- des moyens électroniques (8, 9) pour modifier la gamme de sensibilité du capteur de pression (6) et du photopléthysmographe (17), pour amplifier, enregistrer et/ou afficher les signaux fournis par ceux-ci,
**caractérisé en ce qu'**il comporte en outre:
(i) un réservoir de grand volume (15) rempli d'un fluide incompressible du même type que celui utilisé pour remplir les premier (3) et second (11) réservoirs, et
(ii) une tubulure de pression (10) reliant directement ledit réservoir de grand volume (15) au second réservoir (11), et
(iii) un raccord à trois voies (12) réunissant les tubulures de pression (7) entre le réservoir de grand volume (15) et le second réservoir (11), et dont la troisième voie est obturée par une membrane (13) très fine et souple de type en latex, vis-à-vis de laquelle la sonde de photopléthysmographie (14) est positionnée pour les mesures du pouls artériel pénien.
